# EUROPEAN PATENT SPECIFICATION

(11) **EP 4 329 622 B1**
(45) Date of publication and mention of the grant of the patent: **24.09.2025**
(21) Application number: 23810275.0
(22) Date of filing: 28.06.2023
(51) Int. Cl.: A61B 6/00

(54) **METHODS AND SYSTEMS FOR DETERMINING SCANNING PARAMETERS**
VERFAHREN UND SYSTEME ZUR BESTIMMUNG VON ABTASTPARAMETERN
SYSTÈMES ET PROCÉDÉS POUR LA DÉTERMINATION DE PARAMÈTRES DE BALAYAGE

(30) Priority: 28.06.2022 CN 202210740896
(43) Date of publication of application: 06.03.2024
(73) Proprietor: Shanghai United Imaging Healthcare Co., Ltd., Shanghai 201807 (CN)
(72) Inventor: CUI, Yaying, Shanghai 201807 (CN); LIU, Yangyi, Shanghai 201807 (CN)
(74) Representative: Wang, Bo
(86) International application number: PCT/CN2023/103041
(87) International publication number: WO 2024/002125

(56) References cited:
- CN-A- 105 916 283
- CN-A- 111 631 742
- US-A1- 2007 076 842
- US-A1- 2017 238 896
- US-A1- 2019 038 244
- US-A1- 2019 150 864
- US-A1- 2020 066 007
- US-A1- 2020 330 065
- US-A1- 2020 330 065
- US-A1- 2021 405 228

## Description

### TECHNICAL FIELD

The present disclosure relates to the data processing technical field, in particular, relates to methods and systems for determining scanning parameters of a computer tomography (CT) scan.

### BACKGROUND

A photon-counting computed tomography (PCCT) is widely used in medical imaging due to the advantages in achieving material composition analysis, reducing patient radiation dose, improving CT quantitative analysis accuracy, and achieving ultra-high spatial resolution. Currently, as a new type of computer tomography (CT), the PCCT may not only collect photon numbers through energy bins, but also adjust different thresholds for each bin (i.e., a bin threshold). For PCCT systems, the degree to which the above advantages are achieved depends not only on the appropriate count of bins, the bin threshold, and other energy spectrum parameters, but also on non-energy spectrum parameters such as a tube current, an integration time length corresponding to data in a single field of view, and a gantry speed.

Therefore, it is desired to provide methods for determining scanning parameters of a CT scan, to improve the imaging effect and dose reduction effect of energy spectrum CT.

Document US2020/330065A1 discloses a method for automatically the determining scanning parameters of a photon-counting computed tomography scan.

### SUMMARY

The invention is defined in the independent claims. Particular embodiments are specified in the dependent claims.

### BRIEF DESCRIPTION OF THE DRAWINGS

The present disclosure is further illustrated in terms of exemplary embodiments, and these exemplary embodiments are described in detail with reference to the drawings. These embodiments are not restrictive. In these embodiments, the same number indicates the same structure, wherein:
FIG. 1 is a schematic diagram illustrating application scenario of an exemplary system for determining scanning parameters according to some embodiments of the present disclosure;
FIG. 2 is a block diagram illustrating an exemplary system for determining scanning parameters according to some embodiments of the present disclosure;
FIG. 3 is a flowchart illustrating an exemplary process for determining scanning parameters according to some embodiments of the present disclosure;
FIG. 4 is a flowchart illustrating an exemplary process for determining scanning parameters according to some embodiments of the present disclosure;
FIG. 5 is a flowchart illustrating an exemplary process for determining scanning parameters according to other embodiments of the present disclosure;
FIG. 6 is a flowchart illustrating an exemplary process for determining scanning parameters according to other embodiments of the present disclosure;
FIG. 7 is a block diagram illustrating an exemplary process for determining scanning parameters according to some embodiments of the present disclosure;
FIG. 8 is a flowchart illustrating an exemplary process for determining scanning parameters according to some embodiments of the present disclosure; and
FIG. 9 is a flowchart illustrating an exemplary process for determining scanning parameters according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the accompanying drawing in the following description is merely some examples or embodiments of the present disclosure, for those skilled in the art, the present disclosure may further be applied in other similar situations according to the drawings without any creative effort. Unless obviously obtained from the context or the context illustrates otherwise, the same numeral in the drawings refers to the same structure or operation.

It will be understood that the term "system," "device," "unit," and/or "module" used herein are one method to distinguish different components, elements, parts, sections or assemblies of different levels in ascending order. However, if other words may achieve the same purpose, the words may be replaced by other expressions.

As used in the disclosure and the appended claims, the singular forms "a," "an," and "the" include plural referents unless the content clearly dictates otherwise. Generally speaking, the terms "comprise" and "include" only imply that the clearly identified steps and elements are included, and these steps and elements may not constitute an exclusive list, and the method or device may further include other steps or elements.

The flowcharts used in the present disclosure illustrate operations that the system implements according to the embodiment of the present disclosure. It should be understood that a previous operation or a subsequent operation of the flowcharts may not be accurately implemented in order. Instead, a plurality of steps may be processed in reverse or simultaneously. Moreover, other operations may further be added to these procedures, or one or more steps may be removed from these procedures.

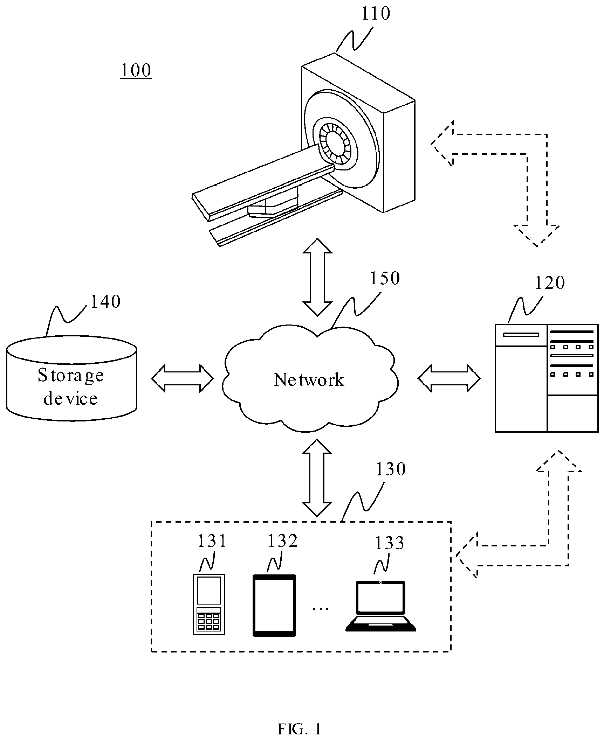

FIG. 1 is a schematic diagram illustrating application scenario of an exemplary system for determining scanning parameters according to some embodiments of the present disclosure.

As shown in FIG. 1, a scanning parameter determination system 100 may include an imaging device 110, a processing device 120, one or more terminals 130, a storage device 140, and a network 150. The components in the system 100 may be connected through one or more connecting manners. Merely for example, as shown in FIG. 1, the imaging device 110 may be connected with the processing device 120 by the network 150. As another example, the imaging device 110 may be directly connected with the processing device 120, for example, the imaging device 110 and the processing device 120 may be connected as indicated by dashed bidirectional arrows in the figure. As a further example, the storage device 140 may be directly connected with the processing device 120 (not shown in FIG. 1) or connected through the network 150. As a further example, the one or more terminals 130 may be directly connected with the processing device 120 (as indicated by the dashed bidirectional arrows connecting the terminal 130 and the processing device 120) or connected through the network 150.

The imaging device 110 may be configured to obtain scanning data of a target object (e.g., a positioning image, a medical image, etc.) by scanning the target object within a detection region. In some embodiments, the target object may include biological and/or non-biological objects. For example, the target object may include specific parts of the body, such as a head, a chest, an abdomen, or a combination thereof. As another example, the target object may be artificial components of a living or inanimate organic and/or inorganic substance. In some embodiments, scanning data related to the target object may include projection data of the target object, one or more scanned images, or the like.

In some embodiments, the imaging device 110 may include non-invasive biological imaging devices for disease diagnosis or research purposes. For example, the imaging device 110 may include a single-mode scanner and/or a multimodal scanner. The single-mode scanner may include an ultrasound scanner, an X-ray scanner, a computed tomography (CT) scanner, a magnetic resonance imaging (MRI) scanner, an ultrasound examiner, a positron emission tomography (PET) scanner, an optical coherence tomography (OCT) scanner, an ultrasound (US) scanner, an intravascular ultrasound (IVUS) scanner, a near-infrared spectroscopy (NIRS) scanner, a far infrared (FIR) scanner, or the like. The multimodal scanner may include an X-ray imaging magnetic resonance imaging (X-ray MRI) scanner, a positron emission tomography X-ray imaging (PET-X-ray) scanner, a single photon emission computed tomography magnetic resonance imaging (SPECT-MRI) scanner, a positron emission tomography-computed tomography (PET-CT) scanner, a digital subtraction angiography magnetic resonance imaging (DSA-MRI) scanner, or the like. The scanner provided above is for illustrative purposes only and is not intended to limit the scope of the present disclosure. As used in this article, the term "imaging modality" or "modality" broadly refers to imaging methods or techniques that collect, generate, process, and/or analyze imaging information of the target object.

In some embodiments, the imaging device 110 may include modules and/or components for performing imaging and/or related analysis. In some embodiments, the imaging device 110 may include a ray generating apparatus, accessory apparatuses, and an imaging apparatus. The ray generation apparatus refers to an apparatus that can generate and control rays (e.g., X-rays). The accessory devices refer to various facilities designed to meet the requirements of clinical diagnosis and treatment, which are matched with the ray generation apparatus. For example, the accessory devices may include mechanical equipment such as examination beds, diagnostic beds, catheter beds, photography beds, various supports, suspension devices, braking devices, filter grids, retention devices, line shields, or the like. In some embodiments, the imaging device 110 may be in various forms, for example, the imaging device 110 may include detectors, computer systems, image processing software, or the like; other imaging devices may include fluorescent screens, film cassettes, image intensifiers, image televisions, or the like.

In some embodiments, data obtained by the imaging device 110 (e.g., medical images of the target object, etc.) may be transmitted to the processing device 120 for further analysis. Additionally or alternatively, the data obtained by imaging device 110 may be sent to the terminal devices (e.g., the terminal 130) for display and/or storage devices (e.g., the storage device 140) for storage.

The processing device 120 may process data and/or information obtained and/or extracted from the imaging device 110, the terminal 130, the storage device 140, and/or other storage devices. For example, the processing device 120 may obtain relevant information of the target object and one or more reference scanning parameters in a scanning parameter set; determine attenuation information of the target object based on the relevant information; and determine one or more target scanning parameters in the scanning parameter set based on the one or more reference scanning parameters and the attenuation information. For example, the processing device 120 may obtain relevant information about the current target object, a scanning protocol, and the target count of the energy bins from the imaging device 110, to determine at least one energy bin threshold based on a tube voltage, attenuation information of a scanning site, the target count of the energy bins, and a preset rule.

In some embodiments, the processing device 120 may be a single server or a group of servers. The server groups may be centralized or distributed. In some embodiments, the processing device 120 may be local or remote. In some embodiments, the processing device 120 may be implemented on a cloud platform. Merely for example, the cloud platform may include private clouds, public clouds, hybrid clouds, community clouds, distributed clouds, internal clouds, multi-layer clouds, or any combination thereof.

In some embodiments, the processing device 120 may be implemented on a computing device. The computing device may be a computer connected with an imaging device (e.g., the imaging device 110), such as a laptop or desktop computer placed in a scanning or operating room. In some embodiments, the processing device 120 may be implemented on a terminal (e.g., the terminal 130). In some embodiments, the processing device 120 may be implemented on an imaging device (e.g., the imaging device 110). For example, the processing device 120 may be integrated into the terminal 130 and/or imaging device 110.

The terminal 130 may be connected with the imaging device 110 and/or processing device 120 for inputting/outputting information and/or data. For example, the user may interact with the imaging device 110 through the terminal 130 to control one or more components of the imaging device 110 (e.g., selecting the scanning protocol, selecting a material to be detected in imaging the target object, setting the target count of energy bins, etc.) As another example, the imaging device 110 may output the generated medical images to the terminal 130 to display to the user.

In some embodiments, the terminal may include a mobile device 131, a tablet computer 132, a laptop computer 133, or any combination thereof. In some embodiments, the mobile device 131 may include a smart home device, a wearable device, a smart mobile device, a virtual reality device, an augmented reality device, or any combination thereof.

In some embodiments, the terminal 130 may recommend and/or display scanning parameters to a user (e.g., doctors, scanning technicians, etc.) via a display interface. For example, the terminal 130 may recommend scanning parameters to the user by displaying a pop-up window containing one or more scanning parameter options and checkboxes for the user by the display interface.

In some embodiments, the one or more terminals 130 may remotely operate the imaging device 110. In some embodiments, the terminal 130 may operate the imaging device 110 via wireless connection. In some embodiments, the one or more terminals 130 may be part of the processing device 120. In some embodiments, the terminal 130 may be omitted.

The storage device 140 may store data and/or instructions. In some embodiments, the storage device 140 may store data obtained from the terminal 130 and/or processing device 120. For example, the storage device 140 may store the target count of energy bins, the scanning protocol, or the like. In some embodiments, the storage device 140 may store data and/or instructions, and the processing device 120 may execute or use the data and/or instructions to execute the exemplary methods described in the present disclosure.

In some embodiments, the storage device 140 may include mass storage devices, removable storage devices, volatile read write memory, read-only memory (ROM), or any combination thereof. The exemplary mass storage may include disks, optical disks, solid-state drives, or the like. The exemplary removable storage may include flash drives, floppy disks, optical disks, memory cards, compressed disks, magnetic tapes, or the like. The exemplary volatile read/write memory may include random access memory (RAM). In some embodiments, storage device 140 may be implemented on a cloud platform. In some embodiments, storage device 140 may be part of the processing device 120.

The network 150 may include any suitable network that can facilitate the exchange of information and/or data of the system 100. In some embodiments, one or more components of the system 100 (e.g., the imaging device 110, the one or more terminals 130, the processing device 120, or the storage device 140) may communicate with one or more other components of the s system 100 to transmit information and/or data. In some embodiments, the network 150 may be any type of wired or wireless network or combination thereof. For example, the network 150 may be and/or include a public network (e.g., the Internet), a private network (e.g., a local area network (LAN), a wide area network (WAN), etc.), a wired network (e.g., an Ethernet), a wireless network (e.g., an 802.11 network, a Wi-Fi network, etc.), a cellular network (e.g., a long-term evolution (LTE) network), a Frame Relay network, a virtual private network ("VPN"), a Satellite Network, a telephone network Routers, hubs, switches, server computers, and/or any combination thereof. In some embodiments, the network 150 may include one or more network access points.

It should be noted that the above description of the system 100 is merely provided for the purposes of illustration, and not intended to limit the scope of the present disclosure. For persons having ordinary skills in the art, multiple variations and modifications for the imaging system and/or the detector may be made under the teachings of the present disclosure. For example, the imaging device 110, the processing device 120, and the terminal 130 may share a storage device 140 or have their own storage devices.

The spectral computed tomography (CT) refers to imaging manners that utilize multi-spectral information to improve the image quality or provide new image information. In a photon-counting computed tomography (PCCT), clinical effects of the spectral CT images (e.g., the image quality, a low contrast resolution, a signal-to-noise ratio, a dose equivalent signal-to-noise ratio, etc.) may depend not only on the selection of the spectral parameters such as the count of the bins (e.g., the count of energy bins), the bin threshold (e.g., an interval size of each of energy bins), or the like, but also depend on non-energy spectral parameters such as a tube voltage, an integral time length corresponding to data in the single field of view, a gantry speed, a pitch, or a scanning time.

Methods and systems for determining scanning parameters may be provided in some embodiments of the present disclosure, at least one energy bin threshold (i.e., the bin threshold) of each of energy bins may be determined based on the scanning protocol and the target count of energy bins. A second-type scanning parameter such as the tube voltage, the integral time length corresponding to data in the single field of view, the gantry speed, the pitch, or the scanning time may be determined based on relevant information of the target object and the scanning parameters such as the target count of energy bins and at least one energy bin threshold. By the methods and systems, the count of energy bins, at least one energy bin threshold, and other spectral parameters may be determined intelligently, efficiently, and reasonably, thereby improving image quality and simplifying workflow; the suitable non-energy spectral parameters may be adaptively determined to optimize clinical imaging results while minimizing scanning dose.

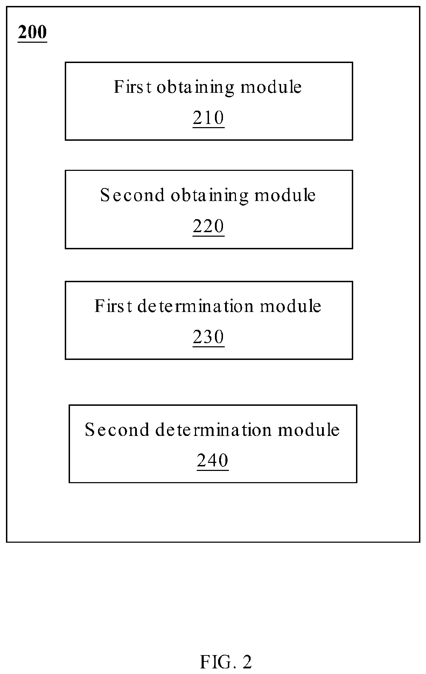

FIG. 2 is a block diagram illustrating an exemplary system for determining scanning parameters according to some embodiments of the present disclosure.

As shown in FIG. 2, in some embodiments, a scanning parameter determination system 200 may include a first obtaining module 210, a second obtaining module 220, a first determination module 230, and a second determination module 240. In some embodiments, the system 200 may be integrated into the imaging device 110 or the processing device 120.

The first obtaining module 210 may be configured to obtain a scanning protocol. In some embodiments, the scanning protocol may include a tube voltage and a scanning site.

The second obtaining module 220 may be configured to obtain a target count of energy bins. In some embodiments, the second obtaining module 220 may be configured to obtain a type of a material to be detected in imaging the target object. In some embodiments, the material may include a material having K-edge effect.

The first determination module 230 may be configured to determine at least one energy bin threshold. In some embodiments, the first determination module 230 may be configured to determine at least one energy bin threshold based on the tube voltage, the scanning site, the target count of energy bins, and a preset rule. In some embodiments, the preset rule may include a difference in counts of photons detected by multiple energy bins being less than a preset threshold.

In some embodiments, the first determination 230 may update at least one energy bin threshold based on the type of energy bins and the preset rule, such that at least one energy bin threshold satisfies the imaging requirements of different base substance maps. In some embodiments, the first determination module 230 may be configured to determine at least one energy bin threshold based on the type of material, the tube voltage, the scanning site, the target count of energy bins, and the preset rule.

In some embodiments, the first determination module 230 may be configured to determine attenuation information of the scanning site of the target object based on relevant information of the target object. In some embodiments, the first determination module 230 may be configured to determine at least one energy bin threshold based on the tube voltage, the attenuation information of the scanning site, the target count of energy bins, and the preset rule.

The second determination module 240 may be configured to determine a second-type scanning parameter. In some embodiments, the second determination module 240 may be configured to determine the second-type scanning parameter based on a first-type scanning parameter and the attenuation information. The first-type scanning parameter may at least include a target count of energy bins (e.g., the target count of energy bins) and at least one energy bin threshold used for imaging the target object. The second-type scanning parameter may include at least one of a tube current, an integral time length corresponding to data in a single field of view, a gantry speed, a pitch, or a scanning time.

In some embodiments, the second determination module 240 may be configured to determine a predicted output of each of energy bins based on the first-type scanning parameter and the attenuation information. The second determination module 240 may also determine the second-type scanning parameter based on the predicted output of each of energy bins, such that the predicted output of the energy bin is substantially consistent with the expected output value and/or the predicted outputs of the energy bins satisfy a preset rule.

It should be noted that the above description of the system 200 is merely provided for the purposes of illustration, and not intended to limit the scope of the present disclosure. For persons having ordinary skills in the art, multiple variations and modifications for the imaging system and/or the detector may be made under the teachings of the present disclosure. However, these modifications and changes are still within the scope of the present disclosure.

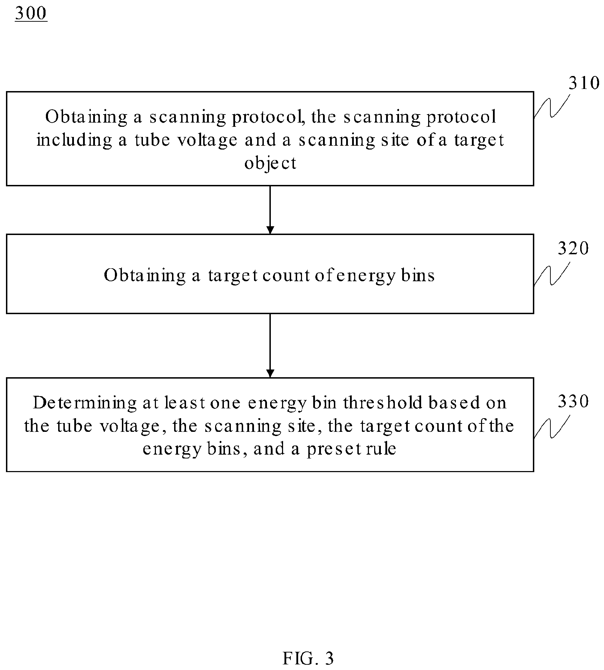

FIG. 3 is a flowchart illustrating an exemplary process for determining scanning parameters according to some embodiments of the present disclosure.

In some embodiments, the process 300 may be performed by the imaging device 110 or the processing device 120. For example, the process 300 may be stored in a storage device (e.g., the storage device 140) in the form of a program or instruction. When the imaging device 110 or processing device 120 executes the program or instruction, the process 300 may be implemented. In some embodiments, the process 300 may be performed by the system 200.

In 310, a scanning protocol may be obtained, wherein the scanning protocol may include a tube voltage and a scanning site of a target object. In some embodiments, the operation 310 may be performed by the processing device 120 or the first obtaining module 210.

The scanning protocol reflects information related to scanning parameters and/or image reconstruction parameters associated with a scan of the target object. For example, the scanning parameters may include a count of scanning layers, a scanning layer thickness, a scanning field of view (FOV), a repetition time (TR), an echo time (TE), an inversion time (TI), an average number of times (NEX), a resolution (e.g., a readout resolution, a phase resolution), a flipping angle (e.g., a flipping angle mode, a minimum flipping angle), or the like. As another example, the image reconstruction parameters may include a reconstruction FOV, a reconstruction slice thickness, a reconstruction matrix, a reconstruction algorithm (e.g., a filtered back projection algorithm, an iterative reconstruction algorithm, etc.), or the like.

In some embodiments, the scanning protocol may include a tube voltage and a scanning site. In some embodiments, relevant information of the scan may be determined based on the scanning protocol, such as the scanning site, the tube voltage, or the like.

During the medical imaging, the rays (e.g., the X-rays) may be emitted from a ray tube ball, which includes a cathode side (e.g., a filament) and an anode side (e.g., a target surface). The tube voltage refers to a voltage applied between the cathode side and anode side of the ray tube, which may be used to form a high-voltage electric field, allowing hot electrons emitted by the filament to rapidly bombard the target surface under the acceleration of the high-voltage electric field and excite the rays. A maximum photon energy of the ray beam generated by the ray tube ball may be equal to a maximum energy of the high-speed electron flow, and the maximum energy of the high-speed electron flow may depend on a peak value of the tube voltage. By changing the tube voltage (also known as a kVp value), the maximum photon energy and ray spectrum may be changed. For example, the tube voltage may be set to 80 kV peak (kVp), 90 kV, or 100 kV, and a detector may be used to scan the target object to obtain scanning data.

In some embodiments, the scanning protocol may be provided by an operator. For example, a doctor or a technician may manually input the scanning protocol corresponding to the target object through an input device of the terminal 130 or imaging device 110. In some embodiments, the scanning protocol may be provided by the system. For example, the system 100 or the imaging device 110 may determine a corresponding scanning protocol based on basic information and historical medical record information of the target object. In some embodiments, the scanning protocol may be selected and determined by the operator. For example, the doctor may determine the scanning protocol for the scan based on the recommended or prestored scanning protocol in the system 100.

In 320, a target count of energy bins may be obtained. In some embodiments, the operation 320 may be performed by the processing device 120 or the second obtaining module 220.

The photon counting detector (PCD) may set different thresholds to determine multiple energy intervals, and count photons in each energy interval, allowing original CT data to contain energy information. The threshold refers to at least one energy bin threshold, and the divided energy interval refers to energy bins. For example, the two energy bins may correspond to energy intervals 20 keV ~60 keV (Electron Volt) and 60 keV ~200 keV. The PCD may not only reduce noise by setting appropriate thresholds, but also collect data from multiple energy intervals simultaneously, making data processing more convenient.

The target count of energy bins reflects a count of energy intervals that need to be divided. In some embodiments, the system may determine the target count of energy bins automatically.

In some embodiments, the target count of energy bins may be determined based on the material to be detected in imaging the target object. Due to the physical properties of the material, when rays with a specific energy is injected into the corresponding material, the material may attenuate the rays, which can easily lead to a lower or even zero photon count value in the corresponding energy interval (i.e., the energy bin). Taking the iodine as an example, due to the physical properties of the iodine, it is relatively difficult to penetrate when the energy value of X-rays exceeds 33 keV. Different materials may correspond to different energy ranges. In some embodiments, the target count of energy bins may be determined based on the count of types of materials. In some embodiments, the target count of energy bins may be automatically determined based on the scanning site. For example, according to different organizational structures of the scanning site, different counts of energy bins may be determined.

In some embodiments, the target count of energy bins may be determined based on a user input. For example, the user may manually input a target count of the energy bins through the input device of the terminal 130 or imaging device 110. In some embodiments, the target count of energy bins may be determined based on user information and/or diagnostic information. For example, a preference of the current physician may be determined based on historical diagnostic data, and the target count of energy bins may be determined based on the preference.

In 330, at least one energy bin threshold may be determined based on the tube voltage, the scanning site, the target count of the energy bins, and a preset rule. In some embodiments, the operation 330 may be performed by the processing device 120 or the first determination module 230.

At least one energy bin threshold also refers to a bin threshold, which can reflect the interval size of each of energy bins. For example, if the target count of energy bins is 2, at least one energy bin threshold may be: E1=20 keV, E2=60 keV, the energy bin 1 may be 20 keV~60 keV, and the energy bin 2 may be 60 keV~a maximum value (i.e., a maximum energy value of the rays or a maximum response value of the detector).

In some embodiments, the preset rule may include that a difference between counts of photons detected by at least two of the energy bins is less than a preset threshold. In some embodiments, the difference may include an absolute value, an average value, a variance, or a mean square error of the difference between counts of photons detected by different energy bins. The preset threshold may be any reasonable value, such as 1, 2, 3, 5, 6, etc. In some embodiments, the preset rule may include a difference value 0 between counts of photons detected by different energy bins. In some embodiments, the preset rule may include that counts of photons detected by different energy bins are basically the same.

In some embodiments, a maximum energy value may be determined based on the tube voltage. According to the maximum energy value, the scanning site, and the target count of energy bins, at least one energy bin threshold that satisfies the preset rule may be determined. In some embodiments, the count of energy bins may be determined based on the target count of energy bins, and based on the maximum energy value, the scanning site, and the target count of energy bins, at least one energy bin threshold that satisfies the preset rule may be determined.

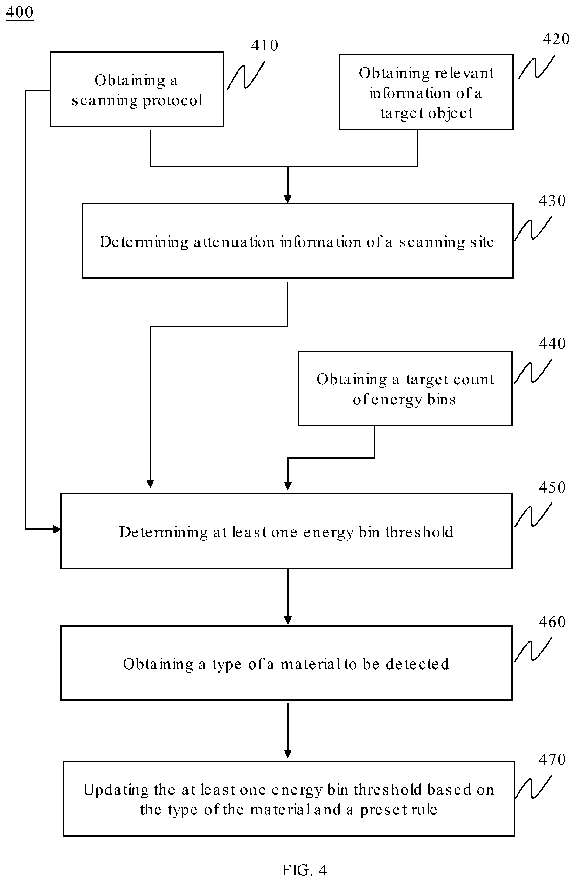

In some embodiments, attenuation information of the scanning site may be determined. Based on the attenuation information, the maximum energy value, the target count of energy bins/the count of energy bins, at least one energy bin threshold that satisfies the preset rule may be determined. For example, relevant information of the target object may be obtained, and the attenuation information of the scanning site of the target object may be determined based on the relevant information. More descriptions of the attenuation information may be found in FIG. 4 (e.g., the operation 430) and related descriptions, which may not be repeated herein.

In some embodiments, a type of the material to be detected in imaging the target object may be obtained, and at least one energy bin threshold that satisfies the preset rule may be determined based on the type of the material to be detected in imaging the target object, the attenuation information, the maximum energy value, the target count of energy bins, so that at least one energy bin threshold may satisfy imaging requirements of different base substance maps. More descriptions of the type of the material to be detected in imaging the target object may be found in FIG. 4 and related descriptions, which may not be repeated herein.

In some embodiments, a spectral map may be determined based on the tube voltage, the scanning site, the target count of energy bins, and at least one energy bin threshold may be determined based on the spectral map. The spectral map reflects photon counting rates corresponding to different energy bins. For example, based on the tube voltage, the scanning site, the target count of energy bins, a spectral map in a coordinate system where the horizontal axis represents the energy keV and the vertical axis represents the photon counting rate (a percentage of photons with a specific energy to total counts of photons) may be determined. By dividing the vertical axis of the spectral map, the data areas corresponding to different energy ranges (corresponding the energy bins) in the graph may be the same, i.e., the photon count values of the energy bins may be the same.

In some embodiments, at least one energy bin threshold may be determined through simulation. For example, a PCCT imaging process of the target object may be simulated based on the scanning site, the maximum energy value, the target count of energy bins, and the relevant information of the target object to determine a spectral shape of the X-ray energy spectrum after passing through the target object and perform threshold partitioning on the obtained spectral shape (e.g., dividing the vertical axis of the spectral map), and further obtain at least one energy bin threshold that ensures that counts of photons detected by each of energy bins is consistent or basically consistent.

In some embodiments, at least one energy bin threshold may be determined based on other feasible approaches. For example, a candidate energy bin threshold may be determined, and predicted counts of photons corresponding to the candidate energy bin threshold may be calculated based on the tube voltage, the scanning site, and the target count of energy bins, to determine at least one energy bin threshold that satisfies the preset rule, which may not be repeated herein.

After the rays are attenuated by the human body, the energy of certain regions of the rays may be absorbed by the human body, causing the energy spectrum to deform. After the energy spectrum is detected by the detector, the energy spectrum may be divided according to the count of bins (e.g., the target count of energy bins), therefore, the tube voltage, the bin threshold, and other factors may cause inconsistent counts of photons for each bin, resulting in inconsistent reconstructed images. By making counts of photons detected by each of energy bins consistent or basically consistent, the reconstructed images with better image quality may be obtained based on detector output data (e.g., counts of photons), thereby improving the accuracy of diagnostic results.

In some embodiments, the determined energy bin threshold may be recommended to the user for selection. For example, the determined energy bin threshold may be outputted and displayed to the user through the display device. In some embodiments, the user may adjust the determined energy bin threshold. In some embodiments, at least one energy bin threshold may be updated based on the user input. In some embodiments, at least one energy bin threshold may be updated based on the type of the material to be detected in imaging the target object and/or the base substance map. For example, at least one energy bin threshold may be updated based on the target base substance map that needs to be reconstructed according to the user's selection, so that at least one energy bin threshold satisfies the imaging requirements of different target base substance maps. As another example, at least one energy bin threshold may be updated based on the type of the material to be detected in imaging the target object selected by the user, such that the reconstructed images obtained based on the updated energy bin threshold satisfy clinical diagnostic requirements (e.g., relevant information of materials having K-edge effect may be observed in the images).

It should be noted that the above description of the process 300 is merely provided for the purposes of illustration, and not intended to limit the scope of the present disclosure. For persons having ordinary skills in the art, multiple variations and modifications of process 300 for the imaging system and/or the detector may be made under the teachings of the present disclosure. However, these modifications and changes are still within the scope of the present disclosure.

FIG. 4 is a flowchart illustrating an exemplary process for determining scanning parameters according to some embodiments of the present disclosure.

As shown in FIG. 4, in some embodiments, attenuation information of the scanning site may be determined based on the relevant information of the target object, and at least one energy bin threshold may be determined based on the attenuation information, the tube voltage, the target count of energy bins, and the preset rule. In some embodiments, the scanning parameter determination process 400 may be executed by the imaging device 110 or the processing device 120. For example, the process 400 may be stored in a storage device (e.g., the storage device 140) in the form of a program or instruction. When the imaging device 110 or processing device 120 executes the program or instruction, the scanning parameter determination method 400 may be implemented. In some embodiments, the process 400 may be performed by the scanning parameters determination system 200.

In 410, a scanning protocol may be obtained. In some embodiments, the operation 410 may be performed by the processing device 120 or the first obtaining module 210.

In some embodiments, the scanning protocol may include a tube voltage and a scanning site. In some embodiments, the tube voltage and the scanning site may be determined based on the scanning protocol. More descriptions may be found in the operation 310 and related descriptions, which may not be repeated herein.

In 420, relevant information of the target object may be obtained. In some embodiments, the operation 420 may be performed by the processing device 120 or the first obtaining module 210.

In some embodiments, the relevant information of the target object may include basic information and image information of the target object. For example, the basic information may include information such as height, weight, body thickness, age, gender, or the like. The image information may include a positioning image and/or camera data of the target object. The positioning image refers to a medical image used to determine a position (e.g., supine, prone, lateral, etc.), a tissue structure, and other information. In some embodiments, the positioning image may include a CT image, a magnetic resonance (MR) image, a digital radiography (DR) image, a positron emission computed tomography (PET) image, etc. The camera data refers to images obtained by camera apparatuses (e.g., a depth camera, etc.). In some embodiments, the positioning image/camera data may include a 2D image, a 3D image, a 4D image, or the like.

In some embodiments, the relevant information of the target object may also include historical diagnostic information related to the target object. For example, the historical diagnostic information may include historical scanned images, historical diagnostic results, historical treatment data, historical scanning protocols, or the like.

In some embodiments, the relevant information of the target object may be obtained from the storage device (e.g., the storage device 140), the imaging device (e.g., the imaging device 110), the medical system (e.g., an internal database of the hospital), or the like.

In 430, attenuation information of the scanning site may be determined. In some embodiments, the operation 430 may be performed by the processing device 120 or the first determination module 230.

The attenuation information reflects an attenuation degree of rays. For example, the attenuation information may be an absorption rate of the X-rays at the scanning site. In some embodiments, the attenuation information of the scanning site may be determined based on the relevant information of the target object and the scanning protocol. For example, the attenuation information of the scanning site may be determined based on the relevant information of the target object and the scan site in the scanning protocol. In some embodiments, the attenuation information of the scanning site may be determined based on the relevant information of the target object.

In some embodiments, the attenuation information may be determined based on the positioning image and the basic information. In some embodiments, the attenuation information may be determined based on the positioning image of the target object. In some embodiments, the attenuation information may be determined based on the basic information of the target object. In some embodiments, the attenuation information may be determined by determining a 3D human model of the target object based on the positioning image and/or the basic information. For example, a 3D human model of the target object may be constructed by determining information of the target object such as structure, density, volume, and layer thickness of organs/tissues, or the like, based on the positioning image and the basic information, and an attenuation coefficient of the rays at the scanning site through the 3D human model may be determined based on the 3D human model. As another example, the 3D human model may be obtained from the database based on the basic information of the target object, and the attenuation coefficient of the rays at the scanning site may be determined through the 3D human model. Different tissue structures and densities have varying attenuation degrees of the rays. In some embodiments, the attenuation information may be determined through data simulation based on a 3D human model of the target object.

In 440, a target count of energy bins may be obtained. In some embodiments, the operation 440 may be performed by the processing device 120 or the second obtaining module 220.

In some embodiments, the target count of energy bins may be determined automatically. In some embodiments, the target count of the energy bins may be determined automatically based on the scanning site of the target object. More descriptions may be found in the operation 320 and related descriptions, which may not be repeated herein.

In 450, at least one energy bin threshold may be determined. In some embodiments, the operation 450 may be performed by the processing device 120 or the first determination module 230.

In some embodiments, at least one energy bin threshold may be determined based on the tube voltage, the attenuation information of the scanning site, and the target count of energy bins. In some embodiments, at least one energy bin threshold that satisfies the preset rule may be determined based on the tube voltage, the attenuation information of the scanning site, and the target count of energy bins. In some embodiments, the spectral map of the target object may be simulated based on the tube voltage, the attenuation information of the scanning site, the target count of energy bins, and at least one energy bin threshold that satisfies the preset rule may be determined based on the spectral map. More descriptions may be found in the operation 330 and related descriptions.

In 460, a type of a material to be detected in imaging the target object may be obtained. In some embodiments, the operation 460 may be performed by the processing device 120 or the second obtaining module 220.

In some embodiments, the material to be detected in imaging the target object may include material having K-edge effect.

The energy of K-edge may be a binding energy of K-layer electron of atom. Due to the absorption of the photon's light band, there is a very large jump in the attenuation coefficient at the K-edge energy of the atom. The material having K-edge effect refers to material with high atomic number that has K-edge absorption and may be used for imaging. In some embodiments, material with K-edge may include iodine (I), gold (Au), rolled (Gd), barium (Ba), ytterbium (Yb), tantalum (Ta), bismuth (Bi), or the like.

In some embodiments, the detected material may be provided by an operator. For example, the doctor or technician may manually input the material to be detected in imaging the target object through the input device of the terminal 130 or imaging device 110. In some embodiments, the material to be detected in imaging the target object may be provided by the system. For example, the system 100 may determine the corresponding material to be detected in imaging the target object based on the basic information and medical record information of the target object. In some embodiments, the material to be detected in imaging the target object may be selected and determined by the operator. For example, the doctor may determine the material to be detected in imaging the target object based on the recommended or pre-stored type of material in the system 100.

In some embodiments, the material to be detected in imaging the target object may include one or more types of materials. In some embodiments, the count of types of materials to be detected may be less than or equal to the target count of energy bins.

In 470, at least one energy bin threshold may be updated based on the type of the material and the preset rule. In some embodiments, the operation 470 may be performed by the processing device 120 or the first determination module 230.

In some embodiments, at least one energy bin threshold may be updated based on the type of material and the preset rule. In some embodiments, an energy value corresponding to the material may be determined according to the type of material, and at least one energy bin threshold may be updated based on the energy value and the preset rule. The energy value corresponding to the material reflects a K-edge absorbed energy of the material. For example, the energy value corresponding to iodine may be 33keV.

By updating at least one energy bin threshold based on the type of the material to be detected in imaging the target object, a better energy bin threshold may be obtained, which can avoid missing data on material having K-edge effects that need to be detected in the detection data (e.g., when the energy value does not correspond to the material, the incident photons may be absorbed by the material, making it impossible to detect and obtain relevant data of the material), and make the data in the reconstructed images more comprehensive and improve the accuracy of diagnostic results.

In some embodiments, at least one energy bin threshold may be updated based on the type of the material to be detected in imaging the target object and the preset rule, such that that at least one energy bin threshold may satisfy the imaging requirements of at least two base substance maps (also referred to as target substance maps).

The ray absorption of a specific substance may be approximately represented by the ray absorption of some other multiple substances (also known as a base substance group). The representation accuracy may be higher if the substance composition of the specific substance is close to the selected base substance group. The images of the base substance group obtained by decomposing the detection data into base materials may be referred to as the base substance maps, also known as the base material maps. For example, the base substance map may include a "water-iodine" map, a "water-calcium" map, a "water-iodine-calcium" map, or the like.

In some embodiments, a target substance map that needs to be reconstructed may be obtained. For example, the base substance group corresponding to the base substance map selected by the user may be obtained. In some embodiments, the material corresponding to the target base substance map and the material having K-edge effect may be the same or different.

In some embodiments, at least one energy bin threshold may be updated based on the type of the material to be detected in imaging the target object and the preset rule, such that at least one energy bin threshold may satisfy the imaging requirements of the target base substance map.

In some embodiments, the imaging requirements of different base substance maps refer to the requirements for the image quality of different base substance maps, such as the requirements for the image resolution, signal-to-noise ratio. In some embodiments, an image quality parameter of a base substance map may satisfy a preset value. For example, the preset value may include values such as image resolution, signal-to-noise ratio set by the user or determined by the system based on diagnostic data (e.g., the scanning site). In some embodiments, the image quality parameters of the base substance map may be optimized. For example, the preset value may be determined based on historical data and statistics so that the generated base substance map has a desired quality to meet diagnosis requirements.

In some embodiments, a machine learning model may be utilized to update at least one energy bin threshold based on the type of material and the preset rule. In some embodiments, at least one energy bin threshold may be updated through data simulation based on the type of material and the preset rule.

It should be noted that the above description of the process 400 is merely provided for the purposes of illustration, and not intended to limit the scope of the present disclosure. For persons having ordinary skills in the art, multiple variations and modifications of process 400 for the imaging system and/or the detector may be made under the teachings of the present disclosure. For example, the operation 440 may be performed to obtain the target count of energy bins, followed by the operation 420 to obtain the relevant information about the target object, and the operation 410 to obtain the scanning protocol. As another example, the operations 440, 410, and 420 may be performed simultaneously. As a further example, the operation 460 may be performed first, and in the operation 450, at least one energy bin threshold may be determined based on the type of material and the attenuation information, the target count of energy bins, or the like. However, these modifications and changes are still within the scope of the present disclosure.

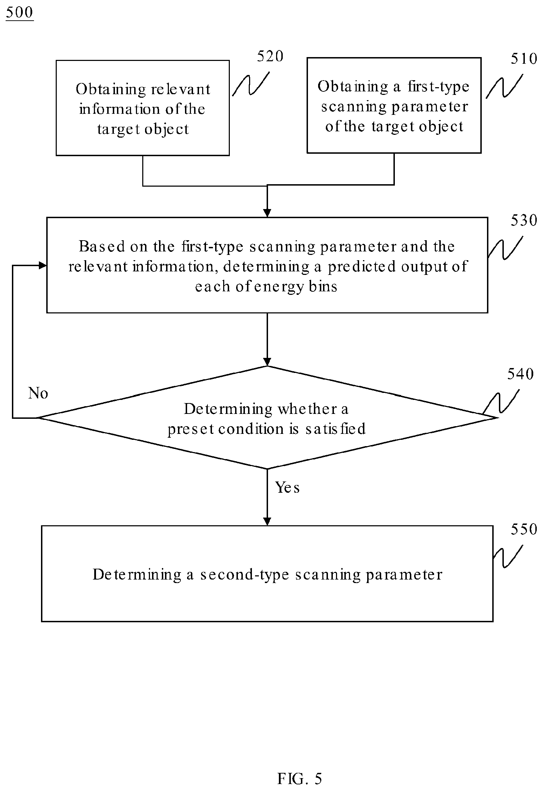

FIG. 5 is a flowchart illustrating an exemplary process for determining scanning parameters according to other embodiments of the present disclosure.

As shown in FIG, 5, in some embodiments, a second-type scanning parameter may be determined based on the relevant information of the target object, the count of energy bins, and at least one energy bin threshold. In some embodiments, the scanning parameter determination process 500 may be performed by the imaging device 110 or the processing device 120. For example, the process 500 may be stored in a storage device (e.g., the storage device 140) in the form of a program or instruction. When the imaging device 110 or processing device 120 executes the program or instruction, the process 500 may be implemented. In some embodiments, the process 500 may be performed by the scanning parameter determination system 200.

In 510, a first-type scanning parameter of the target object may be obtained. In some embodiments, the operation 510 may be performed by the processing device 120, the first obtaining module 210, the second obtaining module 220, or the first determination module 230.

The first-type scanning parameter refers to a parameter directly related to the energy spectrum. For example, the first-type scanning parameter may be an energy value directly related to an energy spectrum curve. The energy spectrum curve may be a curve of the attenuation (i.e., the CT value) of a substance or structure as a function of X-ray energy. From the energy spectrum curve, the average CT value and the standard deviation of each energy point from 40 to 140 keV may be obtained.

In some embodiments, the first-type scanning parameter may include the count of energy bins (i.e., the count of bins) and/or at least one energy bin threshold. For example, the first-type scanning parameter may include the count of energy bins and at least one energy bin threshold determined in the process 300 used for imaging the target object.

In some embodiments, the first-type scanning parameter may be manually inputted by the user. For example, medical staff may choose to input or type at least one energy bin threshold and the count of energy bins corresponding to the current target object through the terminal 130. In some embodiments, the first-type scanning parameter may be automatically determined by the system. For example, the system 100 may determine the corresponding energy bin threshold and the count of energy bins using the process 300 or process 400 based on the scanning protocol and the relevant information of the target object. As another example, the system 100 may determine at least one energy bin threshold and the count of energy bins based on the scanning protocol, the relevant information, and other information of the target object through iterative calculation or a machine learning model. In some embodiments, the first-type scanning parameter may be obtained in any reasonable and feasible way, which may not be limited herein.

In 520, relevant information of the target object may be obtained. In some embodiments, the relevant information of the target object may also include historical data of the target object (e.g., historical diagnostic data, etc.). The operation 520 may be similar to the operation 420 in the process 400, more descriptions may be found in the operation 420, which may not be repeated herein.

In 530, based on the first-type scanning parameter and the relevant information, a predicted output of each of energy bins may be determined. In some embodiments, the operation 530 may be performed by the processing device 120 or the second determination module 240.

The predicted output of each energy bin refers to counts of photons detected by each energy bin. For example, counts of photons of each energy bin output by the photon counting detector may be predicted.

In some embodiments, a predicted output of each energy bin may be determined based on the first-type scanning parameter, the relevant information, and an initial or updated second-type scanning parameter.

The second-type scanning parameter refers to a non-energy spectral parameter that is not directly related to the energy spectrum. In some embodiments, the second-type scanning parameter may include a tube current, an integral time length corresponding to data in a single field of view, a gantry speed, a pitch, a scanning time, or any combination thereof.

The tube voltage (unit: mAs) refers to a current generated by electrons, which are generated by a heated filament and move at high speed towards the anode side under the action of a high-voltage electric field between the anode side and the cathode side. A product of the tube current and the time may determine the count of rays (i.e., counts of emitted photons).

The integral time length corresponding to data in the single field of view refers to the time it takes to collect a single set of data when the gantry rotates during CT imaging. For example, it takes 1 second for the gantry to rotate one circle and collects 9800 sets of data, i.e., 9800 fields of view, the corresponding integral time length for data in the single field of view may be 1/9800 seconds.

The integral time length for data in the single field of view may affect a signal-to-noise ratio of data and counts of photons contained in the data. For example, the longer the integral time length, the more photons the data contains, which may lead to the occurrence of counting saturation phenomenon; the shorter the integral time length, the fewer photons detected, which may lead to the occurrence of hunger effect.

The pitch refers to a moving distance of a scanning table when the gantry rotates one circle.

The combination of the integral time length and pitch corresponding to data under the single field of view may affect the signal-to-noise ratio of the output data of the detector.

The scanning time refers to a total scanning time of the imaging process, such as the duration from a time when the tube begins emitting rays to a time when the tube finishes emitting rays.

The initial second-type scanning parameter refers to a system default value for the second-type scanning parameter that does not take into account factors such as individual differences in the scanning object (i.e., the target object). The updated second-type scanning parameter may be determined by updating or adjusting the initial second-type scanning parameter based on the relevant information, detection requirements, and/or clinical requirements of the target object when the preset condition is not satisfied.

The first-type scanning parameter and the second-type scanning parameter may constitute a complete set of scanning parameters. Based on the complete set of scanning parameters, combined with the relevant information of the target object, the predicted output of each energy bin corresponding to the detector may be obtained.

In some embodiments, the predicted output of each energy bin may be determined using a trained machine learning model based on the first-type scanning parameter, the relevant information of the target object, and the initial/updated second-type scanning parameter. In some embodiments, based on the first-type scanning parameter and the relevant information of the target object, the predicted output of each energy bin may be determined by performing iterative calculations.

In some embodiments, a 3D human model of the target object may be determined based on the relevant information of the target object. The predicted output of each energy bin may be determined based on the 3D human model, the first-type scanning parameter, the relevant information of the target object, and the initial/updated second-type scanning parameter. For example, the 3D human model of the target object may be constructed based on the basic information and a positioning image of the target object. As another example, the 3D human model of the target object may be determined by retrieving a model matching the target object from the database.

The 3D human model reflects organs/tissues contained in the human body, as well as the structure, density, volume, position, and other information of the organs/tissues. In some embodiments, attenuation information of the target object may be calculated based on the 3D human model, and the predicted output of each energy bin may be determined based on the attenuation information, the first-type scanning parameter, and the initial/updated second-type scanning parameter. For example, attenuation information of various tissues/organs of the target object may be calculated based on the 3D human model, and the predicted output of each energy bin may be determined based on the attenuation information, the first-type scanning parameter, and the initial second-type scanning parameter.

In 540, whether a preset condition is satisfied may be determined. In some embodiments, the operation 540 may be performed by the processing device 120 or the second determination module 240.

In some embodiments, whether the predicted output satisfies the preset condition may be determined. When the predicted output satisfies the preset condition, the operation 550 may be performed and the second-type scanning parameter may be determined; otherwise, the initial second-type scanning parameter may be updated until the predicted output satisfies the preset condition.

In some embodiments, the preset condition may include the predicted output of each energy bin being consistent with an expected output, the predicted outputs of the energy bins satisfying the preset rule, etc.

The expected output may be a photon count output of the detector when the image quality satisfies a preset standard. For example, the preset standard may be that the signal-to-noise ratio and the resolution of a reconstructed image are optimal or satisfy diagnostic requirements (e.g., over 80%).

In some embodiments, the expected output may be determined based on requirements on the image quality of a reconstructed image. In some embodiments, the expected output may be determined based on a dose level. For example, different doses may correspond to different expected outputs. In some embodiments, the expected output may be determined based on performance parameters of the detector. For example, the expected output may be determined based on the adaptive working state of the photon counting detector in the CT device. In some embodiments, the expected output may be determined based on any two or more of the image quality requirements, the dose level, the detector performance parameters, or the like.

In some embodiments, the expected output of each energy bin may be determined using a machine learning technique. For example, historical clinical scan images or 3D phantom (i.e., the 3D human model) images, and the corresponding complete set of historical scan parameters (i.e., including a first-type scanning parameter and a second-type scanning parameter) may be used as input to a machine learning model, a mapping relationship between the scanning parameters and image results (e.g., the image quality and dose) may be obtained. Based on the mapping relationship, the expected output of an energy bin corresponding to a specific image quality and/or dose may be determined.

In some embodiments, the expected output of each energy bin may be determined through data simulation. For example, based on prior test results of a large count of 3D human models, a series of mapping curves between o the second-type scanning parameters and the image results under a targeted scanning protocol of various human organs may be obtained, respectively, and the mapping curves may be used for determining the excepted value of each energy bin corresponding to the specific image quality and/or dose.

In some embodiments, the expected outputs corresponding to energy bins determined based on different bin thresholds may be the same or different.

In some embodiments, an interactive operation may be performed such that the predicted output of the energy bin may be consistent with the expected output, and the second-type scanning parameter can be determined. For example, for each energy bin, the predicted output of the energy bin may be compared with the expected output of the energy. When the predicted output of the energy bin is inconsistent with the expected output, one or more of the second-type scanning parameters may be updated based on the mapping curve or the relationship between the scanning parameters and the image results, and the updated second-type scanning parameter may be determined as the final second-type scanning parameter.

Different second-type scanning parameters have different meanings when considering the individual differences of the scanning object (i.e., the target object). For example, for a relatively larger scanning object, the predicted output of each energy bin may be adjusted to be close to the expected output by simply increasing the tube current and keeping other second-type scanning parameters unchanged. As another example, for scanning different human organs, such as the heart, when the heart rate is determined and the heart size is normal, the gantry speed and pitch may be fixed. By comprehensively adjusting the tube current and the corresponding integral time length of the data in the single field of view, the predicted output of each energy bin may be close to the expected output.

In some embodiments, the predicted output may be iteratively updated to satisfy the preset rule to determine the second-type scanning parameter. For example, the iterative updating may be performed based on the predicted output of each energy bin (i.e., the operation 540). When a difference between the predicted output of each energy bin is less than the preset threshold, the preset condition may be satisfied, and the corresponding second-type scanning parameter may be determined as a final second-type scanning parameter. For example, the iterative updating may be performed based on the predicted output of each energy bin (i.e., the operation 540). When the predicted output of each energy bin remains consistent or substantially consistent, the preset condition may be satisfied, and the corresponding second-type scanning parameter may be determined as a final second-type scanning parameter.

In some embodiments, the preset condition may also relate to image quality parameters (e.g., the signal-to-noise ratio, the resolution). In some embodiments, based on the predicted output of each energy bin, the second-type scanning parameter that satisfies a preset image quality parameter may be determined using a trained machine learning model. For example, the predicted output of each energy bin, as well as the preset values corresponding to parameters such as the resolution and the signal-to-noise ratio, may be used as an input of the trained machine learning model to calculate the second-type scanning parameter that satisfies the preset condition.

By determining the second-type scanning parameter based on the relevant information of the target object and the first-type scanning parameter, individual differences in the scanning object may be comprehensively considered to minimize scanning doses while ensuring clinical effectiveness (e.g., the minimum contrast to noise ratio required to observe the current stage of the disease, minimum image quality requirements, etc.).

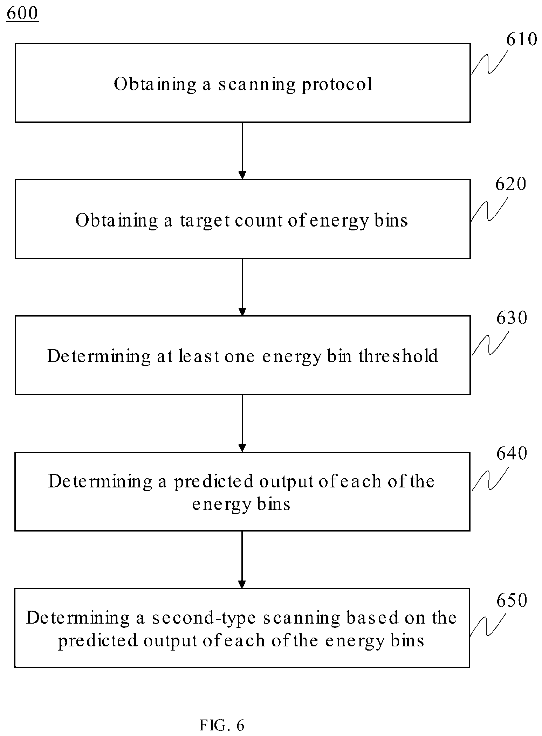

FIG. 6 is a flowchart illustrating an exemplary process for determining scanning parameters according to other embodiments of the present disclosure.

As shown in FIG. 6, in some embodiments, at least one energy bin threshold may be determined based on information such as the relevant information of the target object and the scanning protocol, and the second-type scanning parameter may be further determined based on at least one energy bin threshold, the target count of energy bins, or the like. In some embodiments, a scanning parameter determination process 600 may be executed by the imaging device 110 or the processing device 120. For example, the process 600 may be stored in a storage device (e.g., the storage device 140) in the form of a program or instruction. When the imaging device 110 or processing device 120 executes the program or instruction, the process 600 may be implemented. In some embodiments, the process 600 may be executed by the system 200.

In 610, a scanning protocol may be obtained.

In 620, a target count of energy bins may be obtained.

In 630, at least one energy bin threshold may be determined.

The operations 610, 620, and 630 may be similar to the operations 310, 320, and 330, respectively, more descriptions may be found in the process 300, which may not be repeated herein.

In 640, a predicted output of each of the energy bins may be determined. In some embodiments, the operation 640 may be performed by the processing device 120 or the second determination module 240.

In some embodiments, the predicted output of each energy bin may be determined based on the first-type scanning parameter and the relevant information of the target object. In some embodiments, the first-type scanning parameter may include the target count of energy bins and the at least one energy bin threshold used for imaging the target object. In some embodiments, the predicted output of each energy bin may be determined using a trained machine learning model based on the first-type scanning parameter and the relevant information of the target object. In some embodiments, the predicted output of each energy bin may be determined through multiple iterative calculations based on the first-type scanning parameter and the relevant information of the target object. More descriptions of the predicted output of each energy bin may be found in the operation 530 in the process 500 and related descriptions, which may not be repeated herein.

In 650, a second-type scanning parameter may be determined based on the predicted output of each energy bin. In some embodiments, the operation 650 may be performed by the processing device 120 or the second determination module 240.

In some embodiments, the second-type scanning parameter may be determined based on the predicted output of each energy bin, such that the predicted output of the each of the energy bins is substantially consistent with a corresponding expected output, and/or, the predicted outputs of the energy bins satisfy the preset condition. For example, by performing the operation 530 to the operation 550, the second-type scanning parameter that satisfies the preset condition may be determined. More descriptions may be found in FIG. 5 and related descriptions, which may not be repeated herein. Substantially consistent refers to a difference between the predicted output of the each energy bin and the expected output being less than a difference threshold. The difference threshold may be a system default value, an empirical value, an artificially preset value, or any combination thereof, which can be set according to actual requirements and may not be limited in the present disclosure.

In some embodiments, the imaging device may be controlled to automatically set the relevant parameters based on the determined target count of energy bins, at least one energy bin threshold, and/or the second-type scanning parameter. For example, a control device may control a computer tomography scanner to automatically set parameters based on the target count of energy bins, at least one energy bin threshold, and the second-type scanning parameter.

It should be noted that the above description of the process 500 and process 600 is merely provided for the purposes of illustration, and not intended to limit the scope of the present disclosure. For persons having ordinary skills in the art, multiple variations and modifications of the process 500 and process 600 for the imaging system and/or the detector may be made under the teachings of the present disclosure. However, these modifications and changes are still within the scope of the present disclosure.

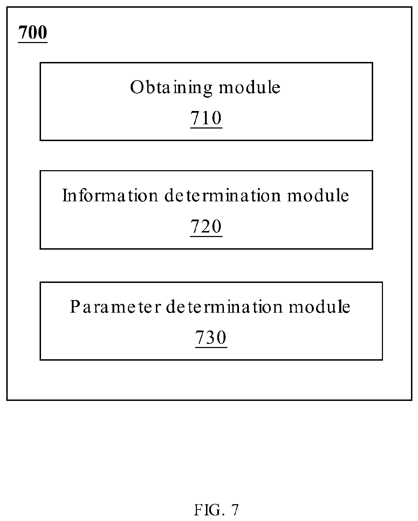

FIG. 7 is a block diagram illustrating an exemplary scanning parameters determination system 700 according to some embodiments of the present disclosure.

As shown in FIG. 7, in some embodiments, the scanning parameters determination system 700 may include an obtaining module 710, an information determination module 720, and a parameter determination module 730. In some embodiments, the system 700 may be integrated into the imaging device 110 or the processing device 120.

The obtaining module 710 may be configured to obtain relevant information of a target object and one or more reference scanning parameters in a scanning parameter set.

In some embodiments, the obtaining module 710 may be configured to obtain a scanning protocol, the scanning protocol may include a tube voltage and a scanning site of the target object; obtain the target count of the energy bins; and determine at least one energy bin threshold based on the tube voltage, the scanning site, the target count of the energy bins, and a preset rule.

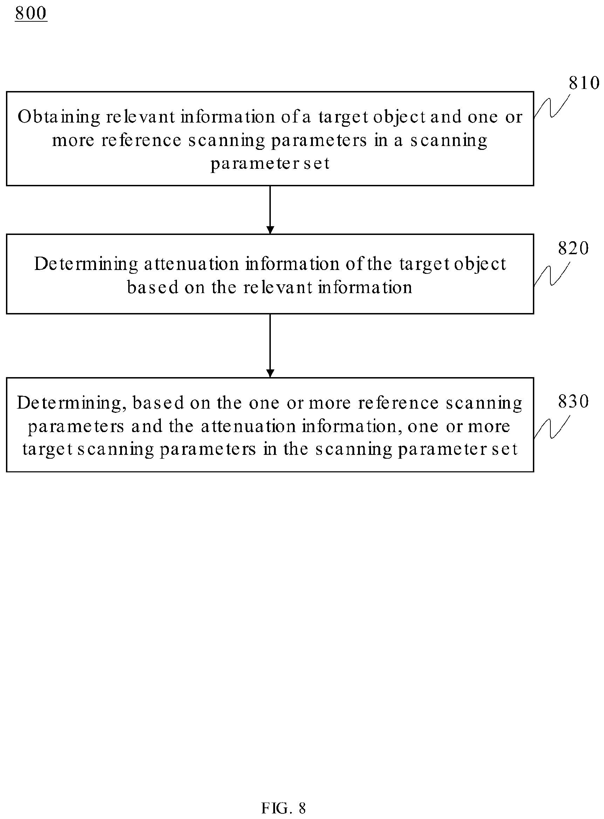

In some embodiments, the obtaining module 710 may be configured to obtain a type of a material to be detected in imaging the target object, the material having K-edge effect; update at least one energy bin threshold according to the type of the material and the preset rule, such that at least one energy bin threshold satisfies imaging requirements of at least two different base material maps. More descriptions of obtaining the relevant information of the target object and the one or more reference scanning parameters in the scanning parameter set may be found in FIG. 8 and related descriptions.

The information determination module 720 may be configured to determine attenuation information of the target object based on the relevant information.

In some embodiments, the information determination module 720 may determine the attenuation information of the target object based on the relevant information and a mapping relationship, the mapping relationship may indicate a relationship between the relevant information and the attenuation information of the target object. More descriptions of determining the attenuation information may be found in FIG. 8 and related descriptions.

The parameter determination module 730 may be configured to determine one or more target scanning parameters in the scanning parameter set based on the one or more scanning parameters and the attenuation information.

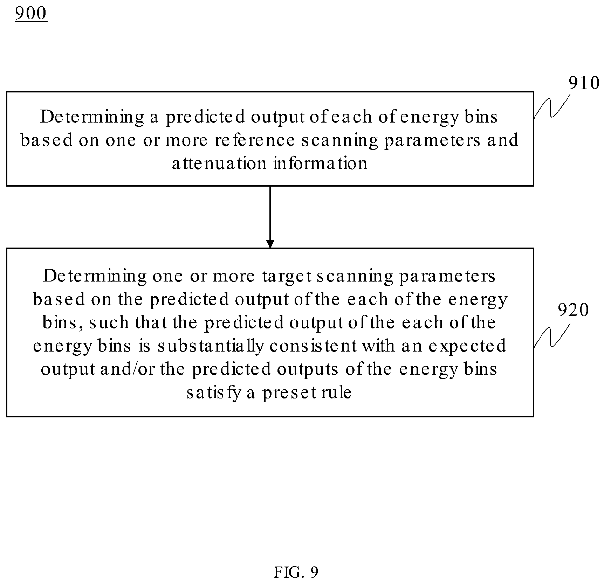

In some embodiments, the parameter determination module 730 may determine a predicted output of each energy bin used for imaging the target object based on the one or more reference scanning parameters and the attenuation information; and determine the one or more target scanning parameters based on the predicted output of each of the energy bins, such that the predicted output of the each of the energy bins is substantially consistent with a corresponding expected output, and/or, the predicted outputs of the energy bins satisfy a preset rule. More descriptions of determining the one or more target scanning parameters in the scanning parameter set may be found in FIG. 9 and related descriptions.

It should be noted that the above description of the scanning parameter determination system 700 is merely provided for the purposes of illustration, and not intended to limit the scope of the present disclosure. It should be understood that, for persons having ordinary skills in the art, after understanding the principle of the system, they may arbitrarily combine various modules or form subsystems connected to other modules without deviating from this principle. However, these modifications and changes are still within the scope of the present disclosure.

FIG. 8 is a flowchart illustrating an exemplary process for determining scanning parameters according to some embodiments of the present disclosure.

In some embodiments, the scanning parameter determination process 800 may be performed by the imaging device 110 or the processing device 120. For example, the process 800 may be stored in the storage device (e.g., the storage device 140) in the form of programs or instructions, when the imaging device 110 or processing device 120 executes the program or instruction, the process 800 may be implemented. In some embodiments, the process 800 may be performed by the scanning parameter determination system 700.

In 810, relevant information of a target object and one or more reference scanning parameters in a scanning parameter set may be obtained.

In some embodiments, the relevant information of the target object may include basic information of the target object and/or image information. In some embodiments, the relevant information may further include historical diagnostic information related to the target object. More descriptions of the relevant information of the target object may be found in FIG. 4 and related descriptions.

In some embodiments, the relevant information of the target object may include information related to a body shape and/or a body fat rate of the target object. For example, the information related to the body shape may include information such as height, weight, body thickness, etc.

In some embodiments, the processing device may obtain the relevant information of the target object from a storage device (e.g., the storage device 140), an imaging device (e.g., the imaging device 110), a medical system, or the like. For example, the body shape and/or the body fat rate of the target object may be obtained from the storage device or the medical system, or determined based on a hospital physical examination result of the target object obtained from the storage device or the medical system.

In some embodiments, the processing device may obtain image data of the target object using a camera device and obtain the relevant information of the target object by processing the image data using image identification manner. The exemplary image identification manner may include but is not limited to a machine learning model, a visual recognition algorithm, or the like. The descriptions of obtaining the relevant information of the target object are merely for illustration, which may not be limited to the embodiments.

The reference scanning parameters refer to parameters related to the imaging quality during the scanning. For example, the reference scanning parameters may include but be not limited to one or more of an integral time length corresponding to data in a single field of view, a gantry speed, a pitch, a scanning time, a layer spacing, a layer thickness, a scanning range, an exposure condition (including a tube voltage, a tube current, an exposure time, etc.), a reconstruction interval, a count of energy bins (i.e., a count of bins), at least one energy bin threshold (i.e., a bin threshold).

In some embodiments, the one or more reference scanning parameters may be preset in advance. For example, a medical staff may choose to input or type the one or more preset scanning parameters corresponding to the current target object through the terminal 130. In some embodiments, the one or more reference scanning parameters may be determined by the system automatically. For example, the system 100 may determine the one or more reference scanning parameters by querying a preset scanning parameter table. The scanning parameter table may be preset based on a relationship between scanning parameters and relevant information of subjects. As another example, the system 100 may determine the one or more reference scanning parameters based on information such as the scanning protocol and the relevant information by performing an iterative calculation and/or using a machine learning model. In some embodiments, the one or more reference scanning parameters may be obtained by any reasonable and feasible way, which may not be repeated herein.

In some embodiments, the one or more reference scanning parameters may include at least one first-type scanning parameter and/or at least one second-type scanning parameter.

In some embodiments, the first-type scanning parameter may be associated with energy used for imaging the target object. For example, the first-type scanning parameter may be an energy value directly related to an energy spectrum curve. In some embodiments, the first-type scanning parameter may includes at least one energy spectrum parameter.

In some embodiments, the first-type scanning parameter may at least include a count of energy bins (i.e., a count of bins) and at least one energy bin threshold (i.e., a bin threshold) used for imaging the target object. For example, the first-type scanning parameter may include a target count of energy bins and/or at least one energy bin threshold determined by the process 300 used for imaging the target object. The count of energy bins may also be referred to as the target count of energy bins.

More description of the first-type scanning parameter, the count of energy bins, at least one energy bin threshold, and the obtaining manners may be found in FIG. 3, FIG. 5, and related descriptions.

The second-type scanning parameter refers to a non-energy spectral parameter that is irrelevant to the energy spectral. For example, the second-type scanning parameter may be a setting parameter of the scanning device, or the like. In some embodiments, the second-type scanning parameter may includes at least one non-energy spectral parameter.

In some embodiments, the second-type scanning parameter may include at least one of a tube current, an integral time length corresponding to data in a single field of view, a gantry speed, a pitch, or a scanning time.

The second-type scanning parameter may be determined by multiple manners. In some embodiments, the second-type scanning parameters may be manually input by the user. In some embodiments, the second-type scanning parameter may be determined by the system.

In some embodiments, the second-type scanning parameter may be determined according to the relevant information of the target object after the first-type scanning parameter is determined. For example, the second-type scanning parameter may be determined based on the first-type scanning parameter and the attenuation information. In some embodiments, the second-type scanning parameter may be obtained by any reasonable and feasible way, which may not be limited herein.

More description of the second-type scanning parameter and the obtaining manner may be found in FIG. 5 and related descriptions.

In some embodiments, the first-type scanning parameter and the second-type scanning parameter may not be divided according to whether it is associated with the energy. In some embodiments, the first-type scanning parameter and the second-type scanning parameter may be divided according to other division rules. For example, the first-type scanning parameter and the second-type scanning parameter may be divided based on whether they are preset. The first-type scanning parameter may be a scanning parameter that can be preset in advance, and the second-type scanning parameter may be further set based on an actual situation of the target object. As another example, the first-type scanning parameter and the second-type scanning parameter may be divided based on whether they need to be updated. The first-type scanning parameter may be a scanning parameter that does not need to be updated, while the second-type scanning parameter may be a scanning parameter that needs to be updated.

In some embodiments, the processing device may obtain a scanning protocol, the scanning protocol including a tube voltage and a scanning site of the target object; obtain the target count of the energy bins; and determine at least one energy bin threshold based on the tube voltage, the scanning site, the target count of the energy bins, and a preset rule, the preset rule including that a difference between counts of photons detected by at least two of the energy bins may be less than a preset threshold. More descriptions of the preset rule, the target count of energy bins, and determining at least one energy bin threshold may be found in FIG. 3 and related descriptions.

In some embodiments, the processing device may obtain a type of a material to be detected in imaging the target object, and update at least one energy bin threshold according to the type of the material and the preset rule, such that at least one energy bin threshold satisfies imaging requirements of at least two different base material maps. More descriptions of the type of the material to be detected in imaging the target object and updating at least one energy bin threshold may be found in FIG. 4 and related descriptions.

In 820, attenuation information of the target object may be determined based on the relevant information.

The attenuation information refers to information that reflects an attenuation degree of rays. For example, the attenuation information may be an absorption rate of X-rays at the scanning site. The attenuation information may be determined in multiple manners.

More descriptions of determining the attenuation information may be found in operation 430 and related descriptions.

In some embodiments, the processing device may determine the attenuation information of the target object based on the relevant information and a mapping relationship, the mapping relationship may indicate a relationship between the relevant information and the attenuation information of the target object. In some embodiments, the relationship between the relevant information and the attenuation information of the target object may be predetermined through simulation experiments. For example, the phantoms with different sizes may be preset for simulating the attenuation of the X-rays to determine the attenuation degrees of different phantoms and reconstruct a mapping relationship between different shapes (e.g., thickness, body fat rate, etc.) and attenuation information of X-rays.

In 830, based on the one or more reference scanning parameters and the attenuation information, one or more target scanning parameters in the scanning parameter set may be determined.

In some embodiments, the target scanning parameters refer to scanning parameters determined according to one or more determined scanning parameters and the attenuation information. In some embodiments, the target scanning parameters refer to scanning parameters that cannot be preset, such as a scanning parameter that needs to be set according to the actual scanning requirements. In some embodiments, the target scanning parameters refer to scanning parameters that need to be updated according to the actual scanning requirements. For example, target scanning parameters may include at least one energy bin threshold that is updated based on the type of the material to be detected in imaging the target object, or the like.

In some embodiments, when the first-type scanning parameter can be preset or predetermined, the target scanning parameters may be a part or all of the second-type scanning parameters, i.e., the second-type scanning parameter may be determined based on the first-type scanning parameters. For example, at least one energy bin threshold may be determined based on the relevant information of the target object and the scanning protocol, and the second-type scanning parameter may be further determined based on at least one energy bin threshold, the target count of energy bins, or the like.

In some embodiments, when the second-type scanning parameter can be preset or predetermined, the target scanning parameters may be a part or all of the first-type scanning parameter, i.e., the first-type scanning parameter may be determined based on the second-type scanning parameters. For example, when performing a heart scan, a gantry speed may be preset and target scanning parameters may be determined based on the set gantry speed to adapt to a heart rate of the target object.

In some embodiments, the processing device 120 may determine one or more target scanning parameters in the scanning parameter set based on the one or more reference scanning parameters. In some embodiments, when the one or more reference scanning parameters include a tube voltage, a scanning site, and a target count of energy bins, the processing device 120 may determine at least one energy bin threshold based on the tube voltage, the scanning site, the target count of energy bins, and a preset rule. At least one energy bin threshold may be a target scanning parameter. In some embodiments, when the one or more reference scanning parameters include a maximum energy value, the scanning site, and the target count of energy bins, the processing device 120 may determine at least one energy bin threshold based on the maximum energy value, the scanning site, the target count of energy bins, and the preset rule.

In some embodiments, the processing device 120 may determine one or more target scanning parameters in the scanning parameter set based on the one or more reference scanning parameters and the attenuation information. In some embodiments, when the one or more reference scanning parameters include a maximum energy value, a target count of energy bins/number of energy bins, the processing device 120 may determine at least one energy bin threshold based on the attenuation information, the maximum energy value, the target count of energy bins/count of energy bins, and the preset rule.

In some embodiments, the processing device 120 may determine one or more target scanning parameters in the scanning parameter set by querying a preset comparison table based on the one or more preset scanning parameters and the attenuation information. The preset comparison table may include corresponding relationships between multiple different reference scanning parameters, multiple different attenuation information, and multiple different target scanning parameters. The preset comparison table may be determined based on data simulation.

In some embodiments, the processing device 120 may determine a predicted output of each energy bin used for imaging the target object based on the one or more reference scanning parameters and the attenuation information; and determine the one or more target scanning parameters based on the predicted output of the each energy bin, such that the predicted output of each energy bin is substantially consistent with a corresponding expected output, and/or, the predicted outputs of the energy bins satisfy a preset rule. More description of the predicted output and determining the one or more target scanning parameters may be found in FIG. 9 and related descriptions.

In some embodiments of the present disclosure, based on the one or more reference scanning parameters and the attenuation information, the one or more target scanning parameters in the scanning parameter set may be determined, which can reasonably determine the one or more scanning parameters, and improve the image quality and reduce the scanning dose.

FIG. 9 is a flowchart illustrating an exemplary process for determining scanning parameters according to some embodiments of the present disclosure.

In some embodiments, a scanning parameter determination process 900 may be performed by the imaging device 110 or the processing device 120. For example, the process 900 may be stored in a storage device (e.g., the storage device 140) in the form of programs or instructions. When the imaging device 110 or processing device 120 executes the program or instruction, the process 900 may be implemented. In some embodiments, the process 900 may be executed by the scanning parameter determination system 700.

In 910, a predicted output of each energy bin may be determined based on the one or more reference scanning parameters and the attenuation information.

The predicted output of each energy bin refers to a count of predicted photons detected by each energy bin. For example, the count of photons of each energy bin output by a photon counting detector may be predicted.

The processing device 120 may determine the predicted output of each energy bin in various manners.

More descriptions of determining the predicted value of each energy bin may be found in the operation 530, the operation 640, and related descriptions.

In 920, one or more target scanning parameters based on the predicted output of each of the energy bins may be determined, such that the predicted output of each of the energy bins is substantially consistent with a corresponding expected output, and/or, the predicted outputs of the energy bins satisfy a preset rule.

More descriptions of the expected output and the determining manner may be found in operation 540 and related descriptions.

The preset rule may be a preset rule or condition used to determine the one or more target scanning parameters. In some embodiments, the preset rule may be used to determine the first-type scanning parameter. In some embodiments, the preset rule may be used to determine the second-type scanning parameter. In some embodiments, the preset rule may be used to determine at least one energy bin threshold.

In some embodiments, the preset rule may include that a difference between counts of photons detected by at least two of the energy bins is less than a preset threshold. In some embodiments, the preset rule may include that a difference between counts of photons detected by at least two of the energy bins is 0. In some embodiments, the preset rule may include that a difference between counts of photons detected by at least two of the energy bins is substantially the same. More descriptions of the preset rule may be found in FIG. 3 and related descriptions.

In some embodiments, the preset rule may be that an image quality parameter (e.g., the signal-to-noise ratio, the resolution, etc.) corresponding to the predicted output reaches a corresponding upper parameter limit when the dose is acceptable.

In some embodiments, the preset rule may be that a signal-to-noise ratio and the resolution of the image corresponding to the predicted output reach a corresponding lower parameter limit under an acceptable image quality.

In some embodiments, the upper and lower parameter limits may be associated with the image quality used for imaging the target object. For example, the upper parameter limit may be an image quality parameter corresponding to the optimal image quality, and the lower parameter limit may be an image quality parameter corresponding to the worst image quality. The optimal image quality refers to the highest image quality that can achieve under an acceptable dose. The worst image quality refers to a lowest image quality that users can accept.

In some embodiments, after the processing device 120 determines the expected output of an energy bin, the terminal device (e.g., the terminal 130) may provide a user with a sliding bar for adjusting the output count of photons of the detector. By receiving an input of the user, the expected output may be adjusted. In some embodiments, the processing device 120 may set a lower limit of parts of the image quality parameters and designate the lower limit of parts of the image quality parameters as a lower limit of the sliding bar during the parameter recommendation process.

In some embodiments, after determining the expected output of each energy bin, the processing device 120 may reconstruct and generate a corresponding simulated image based on the expected output and the target scanning parameters. By presenting a simulated image corresponding to the expected output to the user, the accuracy of the expected output may be confirmed by the user.

In some embodiments, the processing device 120 may determine the one or more target scanning parameters by iteratively updating the predicted output of each energy bin, such that the predicted output of the each of the energy bins is consistent with the expected output. In some embodiments, the processing device 120 may determine the first-type scanning parameter and/or the second-type scanning parameter by iteratively updating the predicted output of each energy bin, such that the predicted output of each energy bin is consistent with the expected output.

In some embodiments, the processing device 120 may determine the one or more target scanning parameters by iteratively updating the predicted output of each energy bin, such that the predicted outputs of the energy bins satisfy the preset rule. In some embodiments, the processing device 120 may determine the first-type scanning parameter and/or the second-type scanning parameter by iteratively updating the predicted output of each energy bin, such that the predicted output of each of the energy bins satisfy the preset rule. More descriptions of determining the target scanning parameters based on the predicted output may be found in FIG. 5, FIG. 6, and related descriptions.

In some embodiments of the present disclosure, by determining the one or more target scanning parameters based on the attenuation information and the one or more scanning parameters, attenuation differences of different scanning objects can be comprehensively considered, and the scanning dose can be reduced while ensuring the acceptable image quality or maximize the image quality while ensuring the acceptable scanning dose.

A scanning parameter determination device may be provided in some embodiments of the present disclosure. In some embodiments, the device may include a scanner (e.g., a computed tomography scanner or a scanner similar to the imaging device 110), a processing device (e.g., a processor similar to the processing device 120), a display device, and a control device. The processing device may be configured to obtain the scanning protocol and the target count of energy bins, and the scanning protocol may include a tube voltage and a scanning site of the target object; and determine at least one energy bin threshold based on the tube voltage, the scanning site of the target object, the target count of energy bins, and the preset rule; and determine a second-type scanning parameter based on the target count of energy bins, at least one energy bin threshold, the relevant information of the target object. The display device may be configured to display the target count of the energy bins, at least one energy bin threshold, and the second-type scanning parameter to the user. The control device may be configured to control a parameter setting of the scanner based on the target count of energy bins, at least one energy bin threshold, and the second-type scanning parameter. More descriptions of determining the scanning parameters may be found elsewhere in the present disclosure, which may not be repeated herein.

The beneficial effects in the embodiments of the present discourse may include but are not limited to: (1) determining at least one energy bin threshold based on the scanning protocol and the target count of energy bins, at least one energy bin threshold may be determined intelligently, efficiently, and reasonably, which can improve image quality and simplify workflows; (2) by ensuring that the difference in counts of photons detected by different energy bins is less than the preset threshold, the energy bins may be determined, ensuring that the image quality and noise corresponding to each of energy bins are basically consistent; (3) by determining the energy bins based on the type of the material to be detected in imaging the target object, the image quality of the base substance map may be guaranteed; (4) by recommending non-energy spectral parameters (e.g., the second-type scanning parameter) based on the energy spectral parameters (e.g., the first-type scanning parameter), the occurrence of detector count saturation and hunger effects can be avoided; (5) by using the relevant information of the target object as parallel input for the first-type scanning parameter, the scanning dose may be minimized while ensuring clinical effectiveness (e.g., a minimum carrier noise ratio (CNR) required for observing the current stage of the disease, minimum image quality requirements, etc.); (6) adaptive determination of dynamic pitch and integral time length corresponding to data in the single field of view may be beneficial for certain specific clinical scanning protocols (e.g., thoracoabdominal continuous scanning) to allocate appropriate non-energy spectral parameters to different scanning sites (i.e., tissues) of the target object, so as to achieve optimal clinical effects while minimizing doses.

In some application scenarios, an imaging may be performed on the object based on the one or more reference scanning parameters and/or the one or more target scanning parameters illustrated above.

It should be noted that different embodiments may produce different beneficial effects. In different embodiments, the possible beneficial effects may be any one or a combination of the above, or any other possible beneficial effects.

The basic concepts have been described. Obviously, for those skilled in the art, the detailed disclosure may be only an example and may not constitute a limitation to the present disclosure. Although not explicitly stated here, those skilled in the art may make various modifications, improvements, and amendments to the present disclosure. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of the specification are not necessarily all referring to the same embodiment. In addition, some features, structures, or features in the present disclosure of one or more embodiments may be appropriately combined.

Moreover, unless otherwise specified in the claims, the sequence of the processing elements and sequences of the present application, the use of digital letters, or other names are not used to define the order of the application flow and methods. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various assemblies described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, this disclosure may not mean that the present disclosure object requires more features than the features mentioned in the claims. In fact, the features of the embodiments are less than all of the features of the individual embodiments disclosed above.

In some embodiments, the numbers expressing quantities, properties, and so forth, used to describe and claim certain embodiments of the application are to be understood as being modified in some instances by the term "about," "approximate," or "substantially." Unless otherwise stated, "about," "approximate," or "substantially" may indicate a ±20% variation of the value it describes. Accordingly, in some embodiments, the numerical parameters set forth in the description and attached claims are approximations that may vary depending upon the desired properties sought to be obtained by a particular embodiment. In some embodiments, the numerical parameters should be construed in light of the number of reported significant digits and by applying ordinary rounding techniques. Although the numerical domains and parameters used in the present application are used to confirm the range of ranges, the settings of this type are as accurate in the feasible range in the feasible range in the specific embodiments.

Each patent, patent application, patent application publication, and other materials cited herein, such as articles, books, instructions, publications, documents, etc., are hereby incorporated by reference in the entirety. In addition to the application history documents that are inconsistent or conflicting with the contents of the present disclosure, the documents that may limit the widest range of the claim of the present disclosure (currently or later attached to this application) are excluded from the present disclosure. It should be noted that if the description, definition, and/or terms used in the appended application of the present disclosure is inconsistent or conflicting with the content described in the present disclosure, the use of the description, definition and/or terms of the present disclosure shall prevail.

At last, it should be understood that the embodiments described in the disclosure are used only to illustrate the principles of the embodiments of this application. Other modifications may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method implemented on at least one machine each of which has at least one processor and at least one storage device for determining scanning parameters of a photon-counting computed tomography scan, comprising:
obtaining relevant information of a target object and one or more reference scanning parameters in a scanning parameter set, the one or more reference scanning parameters including at least one first-type scanning parameter, the first-type scanning parameter at least including a target count of energy bins and at least one energy bin threshold, the obtaining one or more reference scanning parameters in a scanning parameter set including:
obtaining a scanning protocol, the scanning protocol including a tube voltage and a scanning site of the target object;
obtaining the target count of energy bins; and
determining the at least one energy bin threshold based on the tube voltage, the scanning site, the target count of the energy bins, and a preset rule, the preset rule including that a difference between counts of photons detected by different energy bins of the energy bins is less than a preset threshold;
determining attenuation information of the target object based on the relevant information; and
determining one or more target scanning parameters in the scanning parameter set based on the one or more reference scanning parameters and the attenuation information

2. The method of claim 1, wherein the one or more reference scanning parameters further include at least one second-type scanning parameter, the first-type scanning parameter includes at least one energy spectrum parameter, and the second-type scanning parameter includes at least one non-energy spectral parameter.

3. The method of claim 2, wherein the first-type scanning parameter is used for imaging the target object, and the second-type scanning parameter includes at least one of a tube current, an integral time length corresponding to data in a single field of view, a gantry speed, a pitch, or a scanning time.

4. The method of claim 1, wherein the obtaining one or more reference scanning parameters in a scanning parameter set further includes:
obtaining a type of a material to be detected in imaging the target object, the material having K-edge effect.

5. The method of claim 4, wherein the obtaining one or more reference scanning parameters in a scanning parameter set further includes:
updating the at least one energy bin threshold according to the type of the material and the preset rule, such that the at least one energy bin threshold satisfies imaging requirements of at least two different base material maps.

6. The method of any one of claims 1-5, wherein the determining attenuation information of the target object based on the relevant information includes:
determining the attenuation information of the target object based on the relevant information and a mapping relationship, the mapping relationship indicating a relationship between the relevant information and the attenuation information of the target object.

7. The method of any one of claims 1-6, wherein the determining one or more target scanning parameters in the scanning parameter set based on the one or more reference scanning parameters and the attenuation information includes:
determining a predicted output of each of energy bins used for imaging the target object based on the one or more reference scanning parameters and the attenuation information; and
determining the one or more target scanning parameters based on the predicted output of the each of the energy bins, such that the predicted output of the each of the energy bins is substantially consistent with an expected output, and/or, the predicted outputs of the energy bins satisfy a preset rule.

8. The method of any one of claims 1-7, wherein the relevant information includes information related to a body shape, a body thickness, and/or a body fat rate of the target object.

9. An apparatus for determining scanning parameters of a photon-counting computed tomography scan, comprising: a photon-counting computed tomography scanner configured to perform scanning; a processing device configured to implement a method of claims 1 to 8.

## Patentansprüche

1. Verfahren, welches auf zumindest einer Maschine implementiert ist, von denen jede zumindest einen Prozessor und zumindest eine Speichervorrichtung aufweist, zum Bestimmen von Abtastparametern einer photonenzählenden Computertomographie, umfassend:
Erhalten relevanter Informationen über ein Zielobjekt und eines oder mehrerer Referenz-Abtastparameter in einem Abtastparametersatz, wobei der eine oder die mehreren Referenz-Abtastparameter zumindest einen Abtastparameter des ersten Typs beinhalten, wobei der Abtastparameter des ersten Typs zumindest eine Zielanzahl von Energiebehältern und zumindest eine Energiebehälterschwelle beinhaltet, wobei das Erhalten eines oder mehrerer Referenz-Abtastparameter in einem Abtastparametersatz beinhaltet:
Erhalten eines Abtastprotokolls, wobei das Abtastprotokoll eine Röhrenspannung und einen Abtastort des Zielobjekts beinhaltet;
Erhalten der Zielanzahl von Energiebehältern; und
Bestimmen der zumindest einen Energiebehälterschwelle basierend auf der Röhrenspannung, dem Abtastort, der Zielanzahl der Energiebehälter und einer voreingestellten Regel, wobei die voreingestellte Regel beinhaltet, dass ein Unterschied zwischen Anzahlen der von verschiedenen Energiebehältern der Energiebehälter erfassten Photonen kleiner als eine voreingestellte Schwelle ist;
Bestimmen von Dämpfungsinformationen des Zielobjekts basierend auf den relevanten Informationen; und
Bestimmen eines oder mehrerer Ziel-Abtastparameter in dem Abtastparametersatz basierend auf dem einen oder den mehreren Referenz-Abtastparametern und den Dämpfungsinformationen.

2. Verfahren nach Anspruch 1, wobei der eine oder die mehreren Referenz-Abtastparameter weiter zumindest einen Abtastparameter des zweiten Typs beinhalten, der Abtastparameter des ersten Typs zumindest einen Energiespektrumparameter beinhaltet und der Abtastparameter des zweiten Typs zumindest einen nichtenergetischen Spektralparameter beinhaltet.

3. Verfahren nach Anspruch 2, wobei der Abtastparameter des ersten Typs zum Abbilden des Zielobjekts verwendet wird und der Abtastparameter des zweiten Typs zumindest eines von einem Röhrenstrom, einer integralen Zeitlänge, welche den Daten in einem einzelnen Sichtfeld entspricht, einer Gantry-Geschwindigkeit, einer Neigung oder einer Abtastzeit beinhaltet.

4. Verfahren nach Anspruch 1, wobei das Erhalten eines oder mehrerer Referenz-Abtastparameter in einem Abtastparametersatz weiter beinhaltet:
Erhalten einer Materialart, welche beim Abbilden des Zielobjekts erfasst werden soll, wobei das Material einen K-Kanten-Effekt aufweist.

5. Verfahren nach Anspruch 4, wobei das Erhalten eines oder mehrerer Referenz-Abtastparameter in einem Abtastparametersatz weiter beinhaltet:
Aktualisieren der zumindest einen Energiebehälterschwelle entsprechend der Art des Materials und der voreingestellten Regel, sodass die zumindest eine Energiebehälterschwelle Abbildungsanforderungen von zumindest zwei verschiedenen Basismaterialkarten erfüllt.

6. Verfahren nach einem der Ansprüche 1-5, wobei das Bestimmen von Dämpfungsinformationen des Zielobjekts basierend auf den relevanten Informationen beinhaltet:
Bestimmen der Dämpfungsinformationen des Zielobjekts basierend auf den relevanten Informationen und einer Zuordnungsbeziehung, wobei die Zuordnungsbeziehung eine Beziehung zwischen den relevanten Informationen und den Dämpfungsinformationen des Zielobjekts angibt.

7. Verfahren nach einem der Ansprüche 1-6, wobei das Bestimmen eines oder mehrerer Ziel-Abtastparameter in dem Abtastparametersatz basierend auf dem einen oder den mehreren Referenz-Abtastparametern und den Dämpfungsinformationen beinhaltet:
Bestimmen einer vorhergesagten Ausgabe jeder der Energiebehälter, welche zum Abbilden des Zielobjekts verwendet werden, basierend auf dem einen oder den mehreren Referenz-Abtastparametern und den Dämpfungsinformationen; und
Bestimmen des einen oder der mehreren Ziel-Abtastparameter basierend auf der vorhergesagten Ausgabe jeder der Energiebehälter, sodass die vorhergesagte Ausgabe jeder der Energiebehälter im Wesentlichen mit einer erwarteten Ausgabe übereinstimmt, und/oder die vorhergesagten Ausgaben der Energiebehälter eine voreingestellte Regel erfüllen.

8. Verfahren nach einem der Ansprüche 1-7, wobei die relevanten Informationen Informationen bezüglich einer Körperform, einer Körperdicke und/oder eines Körperfettanteils des Zielobjekts beinhalten.

9. Einrichtung zum Bestimmen von Abtastparametern einer photonenzählenden Computertomographie, umfassend:
einen photonenzählenden Computertomographen, welcher zum Durchführen von Abtastungen konfiguriert ist;
eine Verarbeitungsvorrichtung, welche zum Implementieren eines Verfahrens nach Ansprüchen 1 bis 8 konfiguriert ist.

## Revendications

1. Procédé mis en œuvre sur au moins une machine qui présente chacune au moins un processeur et au moins un dispositif de stockage pour déterminer des paramètres de balayage d'une tomodensitométrie à comptage photonique, comprenant :
l'obtention d'informations pertinentes sur un objet cible et d'un ou de plusieurs paramètres de balayage de référence dans un ensemble de paramètres de balayage, les un ou plusieurs paramètres de balayage de référence incluant au moins un paramètre de balayage de premier type, le paramètre de balayage de premier type incluant au moins un nombre cible de plages d'énergie et au moins un seuil de plage d'énergie, l'obtention d'un ou de plusieurs paramètres de balayage de référence dans un ensemble de paramètres de balayage incluant :
l'obtention d'un protocole de balayage, le protocole de balayage incluant une tension de tube et un site de balayage de l'objet cible ;
l'obtention du nombre cible de plages d'énergie ; et
la détermination du au moins une seuil de plage d'énergie sur la base de la tension de tube, du site de balayage, du nombre cible des plages d'énergie et d'une règle prédéfinie, la règle prédéfinie incluant le fait qu'une différence entre les nombres de photons détectés par différentes plages d'énergie parmi les plages d'énergie est inférieure à un seuil prédéfini ;
la détermination d'informations d'atténuation de l'objet cible sur la base des informations pertinentes ; et
la détermination d'un ou de plusieurs paramètres de balayage cibles dans l'ensemble de paramètres de balayage sur la base des un ou plusieurs paramètres de balayage de référence et des informations d'atténuation.

2. Procédé selon la revendication 1, dans lequel les un ou plusieurs paramètres de balayage de référence incluent en outre au moins un paramètre de balayage de second type, le paramètre de balayage de premier type inclut au moins un paramètre de spectre d'énergie, et le paramètre de balayage de second type inclut au moins un paramètre spectral non énergétique.

3. Procédé selon la revendication 2, dans lequel le paramètre de balayage de premier type est utilisé pour imager l'objet cible et le paramètre de balayage de second type inclut au moins un d'un courant de tube, d'une durée intégrale correspondant à des données dans un seul champ de vision, d'une vitesse de portique, d'un pas et d'un temps de balayage.

4. Procédé selon la revendication 1, dans lequel l'obtention d'un ou de plusieurs paramètres de balayage de référence dans un ensemble de paramètres de balayage inclut en outre :
l'obtention d'un type d'un matériau à détecter dans l'imagerie de l'objet cible, le matériau présentant un effet K-edge.

5. Procédé selon la revendication 4, dans lequel l'obtention d'un ou de plusieurs paramètres de balayage de référence dans un ensemble de paramètres de balayage inclut en outre :
la mise à jour du au moins un seuil de plage d'énergie en fonction du type du matériau et de la règle prédéfinie, de telle sorte que le au moins un seuil de plage d'énergie satisfasse à des exigences d'imagerie d'au moins deux différentes cartes de matériaux de base.

6. Procédé selon l'une quelconque des revendications 1-5, dans lequel la détermination d'informations d'atténuation de l'objet cible sur la base des informations pertinentes inclut :
la détermination des informations d'atténuation de l'objet cible sur la base des informations pertinentes et d'une relation de mappage, la relation de mappage indiquant une relation entre les informations pertinentes et les informations d'atténuation de l'objet cible.

7. Procédé selon l'une quelconque des revendications 1-6, dans lequel la détermination d'un ou de plusieurs paramètres de balayage cibles dans l'ensemble de paramètres de balayage sur la base des un ou plusieurs paramètres de balayage de référence et des informations d'atténuation inclut :
la détermination d'une sortie prédite de chacune des plages d'énergie utilisées pour imager l'objet cible sur la base des un ou plusieurs paramètres de balayage de référence et des informations d'atténuation ; et
la détermination des un ou plusieurs paramètres de balayage cibles sur la base de la sortie prédite de la chacune des plages d'énergie, de telle sorte que la sortie prédite de la chacune des plages d'énergie soit sensiblement cohérente avec une sortie attendue, et/ou que les sorties prédites des plages d'énergie satisfassent une règle prédéfinie.

8. Procédé selon l'une quelconque des revendications 1-7, dans lequel les informations pertinentes incluent des informations relatives à une forme de corps, une épaisseur de corps et/ou un taux de graisse corporelle de l'objet cible.

9. Appareil pour déterminer des paramètres de balayage d'une tomodensitométrie à comptage photonique, comprenant :
un tomodensitomètre à comptage photonique configuré pour réaliser un balayage ;
un dispositif de traitement configuré pour mettre en œuvre un procédé selon les revendications 1 à 8.
